# EUROPEAN PATENT SPECIFICATION

(11) **EP 2 214 634 B1**
(45) Date of publication and mention of the grant of the patent: **30.04.2014**
(21) Application number: 08848952.1
(22) Date of filing: 13.11.2008
(51) Int. Cl.: A61K 8/97, A61K 36/54, A61P 9/00, A61P 17/16, A61K 8/02, A61K 8/06, A61Q 19/10, A61Q 5/06, A61Q 5/00, A61Q 19/00

(54) **EXTERNAL PREPARATIONS FOR MOSTURIZING THE SKIN CONTAINING LITSEA POLYANTHA**
EXTERNE FEUCHTIGKEITSSPENDENDE ZUBEREITUNGEN FÜR DIE HAUT AUF BASIS VON LITSEA POLYANTHA
PRÉPARATIONS EXTERNES HYDRATANTES POUR LA PEAU A BASE DE LITSEA POLYANTHA

(30) Priority: 15.11.2007 JP 2007297334
(43) Date of publication of application: 11.08.2010
(73) Proprietor: Anbas Corporation, Kita-ku Osaka-shi Osaka 531-0072 (JP); Lohajoti, Poonsook, Nan 55000 (TH)
(72) Inventor: LOHAJOTI, Poonsook, 55000 (TH); NAGAI, Katsuya, Kita-ku, Osaka-shi Osaka 531-0072 (JP); TANIDA, Mamoru, Kita-ku, Osaka-shi Osaka 531-0072 (JP); SHEN, Jiao, Kita-ku, Osaka-shi Osaka 531-0072 (JP); HORII, Yuko, Kita-ku, Osaka-shi Osaka 531-0072 (JP)
(74) Representative: Müller-Boré & Partner Patentanwälte PartG mbB
(86) International application number: PCT/JP2008/071064
(87) International publication number: WO 2009/064023

(56) References cited:
- JP-A- 9 087 197
- JP-A- 2000 256 142
- JP-A- 2001 031 528
- JP-A- 2001 226 218
- JP-A- 2001 226 218
- JP-A- 2006 265 141
- JP-A- 2006 265 141
- RAHMAN A ET AL: "Comparison of rhizosphere and non-rhizosphere microflora of som (Machilus bombycina) and Soalu (Litsea polyantha) plants growing in Goalpara district, Assam, India", MEDICINAL & AROMATIC PLANTS ABSTRACTS, SCIENTIFIC PUBLISHERS, SCIENTIFIC PUBLISHERS, NEW DELHI - INDIA, vol. 25, no. 6, 1 December 2003 (2003-12-01), XP018015339, ISSN: 0250-4367
- POONIA ET AL: "Anti-diarrheal activity of methanol extract of Litsea polyantha bark in mice", FITOTERAPIA, IDB HOLDING, MILAN, IT, vol. 78, no. 3, 13 April 2007 (2007-04-13) , pages 171-174, XP022030650, ISSN: 0367-326X, DOI: 10.1016/J.FITOTE.2006.10.004
- BANERJI N ET AL: "An arabinoxylan from the mucilage of the leaves of Litsea polyantha", CARBOHYDRATE RESEARCH, PERGAMON, GB, vol. 147, no. 1, 1 March 1986 (1986-03-01) , pages 165-168, XP026638325, ISSN: 0008-6215, DOI: 10.1016/0008-6215(86)85017-0 [retrieved on 1986-03-01]
- CHOTANI: "Silent Î 2C-adrenergic receptors enable cold-induced vasoconstriction in cutaneous arteries", AJP: HEART AND CIRCULATORY PHYSIOLOGY, vol. 278, no. 4, 1 January 2000 (2000-01-01), page H1075, XP55038469, ISSN: 0363-6135
- CHARKOUDIAN NISHA: "Skin blood flow in adult human thermoregulation: how it works, when it does not, and why.", MAYO CLINIC PROCEEDINGS. MAYO CLINIC MAY 2003 LNKD- PUBMED:12744548, vol. 78, no. 5, May 2003 (2003-05), pages 603-612, XP002683794, ISSN: 0025-6196
- LINDBLAD L E ET AL: "Neural regulation of vascular tone and cold induced vasoconstriction in human finger skin", JOURNAL OF THE AUTONOMIC NERVOUS SYSTEMS, ELSEVIER, AMSTERDAM, NL, vol. 30, no. 2, 1 June 1990 (1990-06-01), pages 169-173, XP024531958, ISSN: 0165-1838, DOI: 10.1016/0165-1838(90)90141-5 [retrieved on 1990-06-01]

## Description

The present invention relates to a skin moisturizer and a blood circulation promoter. The present invention further relates to external preparations for the skin comprising the skin moisturizer or the blood circulation promoter and thus having excellent moisturizing effects or blood circulation-promoting effects.

The water content of the horny layer of the skin is closely connected with the condition of the skin. To maintain healthy bare skin, an appropriate amount of water needs to be retained in the horny layer. It is known that natural moisturizing factors (NMF) greatly contribute to the water retention of the horny layer (see Non-patent Document 1). In particular, amino acids, which are the main components of NMF, are considered to be deeply involved in the water retention of the horny layer. However, it has been reported that the amount of amino acids in the horny layer decreases with age or is reduced due to the external environment, which results in reduced water retention function of the horny layer (see Non-Patent Documents 2 and 3). This reduction in the water retention function of the horny layer is one of the factors that cause wrinkles to be formed because of reduced skin moisture and reduced elasticity with age.

To improve such water retention function and enhance the moisture retention of the skin, various types of moisturizers (such as glycerin, propylene glycol, sorbit, trehalose, 1,3-butylene glycol, hyaluronic acid, and sodium salts thereof) have been developed, and made commercially available. By application to the skin, such moisturizers inhibit transepidermal water loss (TEWL) and retain the water content of the skin, thus providing skin moisturizing effects. Urea is one of the components of NMF, and is widely used as a moisturizer. As part of the moisturizing mechanism of urea in the horny layer, "hydrogen bonding" is considered to retain water in the horny layer and prevent the skin from drying.

There have been various natural product-derived components proposed for use as moisturizers, such as plant extracts and bacteria cell extracts (see, for example, Patent Documents 1 to 6). Patent Documents 1 to 6 specifically describe that extracts of banyan and plants of the families Cunoniaceae, Welwitschiaceae, Hydrophyllaceae and Fouquieriaceae are effective as moisturizers and bacteria cell activators.

Patent Document 7 describes that extracts of plants belonging to the genus Blechnum of the family Blechnaceae of the order Filicales and plants belonging the genus Polystichum of the family Dryopteridaceae have cell-activating properties and are effective as antioxidants.
Non-Patent Document 1: Tetsuji Hirao, "Current NMF Research", Fragrance J., 17 (2000)
Non-Patent Document 2: Yoshiyuki Kono, "Utility of Skin-Moisturizing and Rough Skin-Preventing Cosmetics, and the Development of Products", J. Soc. Cosmet. Chem. Japan 36 (4) (2002)
Non-Patent Document 3: Hitoshi Masaki et al., "Keratin Moisture and Aging", Fragrance J., 8 (1993)
Patent Document 1: Japanese Unexamined Patent Publication No. 1996-208427
Patent Document 2: Japanese Unexamined Patent Publication No. 2007-238455
Patent Document 3: Japanese Unexamined Patent Publication No. 2007-246489
Patent Document 4: Japanese Unexamined Patent Publication No. 2007-246490
Patent Document 5: Japanese Unexamined Patent Publication No. 2007-246491
Patent Document 6: Japanese Unexamined Patent Publication No. 2007-246492
Patent Document 7: Japanese Unexamined Patent Publication No. 2003-321377

Many attempts have been made to utilize plant extracts as moisturizers or cell activators, as described above. However, such moisturizers and cell activators do not have sufficient effects, and are far from being completely satisfactory. Thus, an object of the present invention is to find a natural-product component having excellent moisturizing effects and a natural-product component capable of promoting blood flow to activate cells, and provide a skin moisturizer and a blood circulation promoter each utilizing such an excellent component. Another object is to provide external preparations for the skin utilizing the moisturizing effects of the skin moisturizer and the blood circulation-promoting effects (blood flow-enhancing effect) of the blood circulation promoter.

To achieve the above objects, the present inventors conducted extensive research, and found that Litsea polyantha has excellent skin-moisturizing effects and blood circulation-promoting effects. As a result of this research, the inventors confirmed that pulverized leaves or a lipid-soluble fraction of a leaf extract of Litsea polyantha can be used as an active ingredient of external preparations for the skin that have moisturizing effects and blood circulation-promoting effects. The present invention has been accomplished, based on this finding.

Thus, the present invention provides the following items:
(I) Skin Moisturizer
   (I-1) A skin moisturizer comprising, as an active ingredient, pulverized leaves or a lipid-soluble fraction of a leaf extract of Litsea polyantha in an amount effective to reduce the sympathetic nerve activity of a cutaneous artery.
(II) Blood Circulation Promoter
   (II-1) A blood circulation promoter comprising, as an active ingredient, pulverized leaves or a lipid-soluble fraction of a leaf extract of Litsea polyantha in an amount effective to reduce the sympathetic nerve activity of a cutaneous artery.
(III) External Skin Preparation
   (III-1) An external preparation for the skin comprising, as an active ingredient, pulverized leaves or a lipid-soluble fraction of a leaf extract of Litsea polyantha in an amount effective to reduce the sympathetic nerve activity of a cutaneous artery.
   (III-6) An external preparation for the skin containing a skin moisturizer described in (I-1) or a blood circulation promoter described in (II-1) in an amount effective to reduce the sympathetic nerve activity of a cutaneous artery.

The present invention can provide a skin moisturizer having excellent moisturizing effects, and provide a blood circulation promoter having blood flow-promoting effects. The skin moisturizer can be effectively used as a moisturizing component of external preparations for the skin that have moisturizing effects (such as cosmetics and external pharmaceuticals), particularly cosmetics. The blood circulation promoter can be used as a blood circulation-promoting component of external preparations for the skin that have blood flow-promoting effects (such as external pharmaceuticals and cosmetics).

The present invention can provide external preparations for the skin (such as cosmetics and external pharmaceuticals) containing the above skin moisturizer as an active ingredient and thus having excellent moisturizing effects. Further, the present invention can provide external preparations for the skin (such as cosmetics and external pharmaceuticals) containing the above blood circulation promoter as an active ingredient and thus promoting blood flow in the face, head, limbs or the whole body to prevent and ameliorate disorders (such as skin dullness, age spots, wrinkles, hair loss, and thinning hair due to reduced metabolism) and pathologic conditions (such as suffering from sensitivity to cold due to poor circulation, stiff shoulders associated with poor circulation, congestion, bedsores, Raynaud's disease, and economy-class syndrome), all caused by blood circulation disorders.

### (I) Processed Product of a Plant Belonging to the Genus Litsea of the Family Lauraceae

According to the present invention, pulverized leaves or a lipid-soluble fraction of a leaf extract of Litsea polyantha is/are used. Generally, any plant that belongs to the genus Litsea of the family Lauraceae can be used for a skin moisturizer, a blood circulation promoter or an external preparation. Specific examples thereof include Litsea acuminata, Litsea lancifolia (Sieb. et Zucc.) F. Vills., Litsea japonica, Litsea calicaris, Litsea cubeba, Litsea ichangensis, Litsea citrata, Litsea polyantha, and the like. Litsea polyantha is used according to the present invention.

Generally, any portion of the above plants can be used for a skin moisturizer, a blood circulation promoter or an external preparation. The whole plant or a part of the plant (such as leaves, flowers, seeds, roots, stems, and buds) may be used. Generally, the leaves, roots, stems, barks, and seeds are preferable from the viewpoint of the ease of availability. Generally using the leaves or seeds, particularly leaves, is preferable from the viewpoint of efficacy.

General examples of the processed products include crushed products, pulverized products, juices, and extracts of the whole plant or a part of the plant. Generally, such crushed products or pulverized products may be used as is or after being dried.

The whole plant or a part of the plant, as is or after being dried, may be subjected to solvent extraction. Generally, considering extraction efficiency, the whole plant or a part of the plant is preferably subjected to extraction after processing such as slicing and pulverization. The extraction may be performed by immersion in an extractant or by supercritical fluid extraction or subcritical fluid extraction. Stirring or homogenization in the extractant may be performed to enhance extraction efficiency. The extraction temperature is not particularly limited. For example, a temperature in the range of about 5°C to a temperature not higher than the boiling point of the extractant may be used. The extraction time may vary according to the type of extractant and the extraction temperature used. The extraction time is usually about 1 hour to about 14 days.

General examples of extractants include water; lower alcohols such as methanol, ethanol, propanol, and isopropanol; polyhydric alcohols such as 1,3-butylene glycol, propylene glycol, and dipropylene glycol, and glycerin; ethers such as ethyl ether, and propyl ether; esters such as butyl acetate, and ethyl acetate; ketones such as acetone, and ethyl methyl ketone; chloroform, dichloromethane, and like organic solvents. According to the present invention, organic solvents that are capable of extracting a lipid-soluble fraction from the leaves of Litsea polyantha are preferably used. The extractants may be used singly or as a mixture of two or more thereof. Examples of such solvent mixtures include a mixture of chloroform with methanol or ethanol. One or more kinds of supercritical fluids or subcritical fluids such as carbon dioxide, ethylene, propylene, ethanol, methanol, and ammonia may be used.

Generally, the thus obtained solvent extract of a plant of the genus Litsea may be used for a skin moisturizer, a blood circulation promoter or an external preparation as it is, or the extract may further be concentrated or dried, dissolved again in a nonpolar solvent, and used. As long as the effects of the present invention are not impaired, the leaf extract may be subjected to purification, such as decolorization, deodorization, and desalting, or fractionated by column chromatography, etc., and the obtained fraction may be used for the invention. The lipid-soluble fraction of a leaf extract of Litsea polyantha may be lyophilized, and dissolved when used.

### (II) Skin Moisturizer

The pulverized leaves or a lipid-soluble fraction of a leaf extract of Litsea polyantha has excellent moisturizing effects as shown in Experiment 1 below, and therefore can be used as an active ingredient of skin moisturizers, particularly skin moisturizers in an epidermally administrable form. More specifically, the present invention can provide a skin moisturizer in an epidermally administrable form containing pulverized leaves or a lipid-soluble fraction of a leaf extract of Litsea polyantha as an active ingredient.

The proportion of pulverized leaves or a lipid-soluble fraction of a leaf extract of Litsea polyantha in the skin moisturizer of the invention is not particularly limited, as long as the obtained skin moisturizer has moisturizing effects. The proportion can be appropriately selected from the range of 0.1 to 100 wt.%, preferably 1 to 100 wt.%, and more preferably 2.5 to 100 wt.%, based on the total weight.

The form of the moisturizer is not particularly limited, as long as the moisturizer is epidermally administrable. Examples of such forms include liquids, emulsions, creams, ointments, gels, aerosols, and patches such as packs.

The skin moisturizer of the invention can be used as an active ingredient of external preparations for the skin having moisturizing effects. Such an external preparation for the skin contains the skin moisturizer as an essential ingredient and may further contain other components usually contained in external preparations for the skin (such as drugs, quasi drugs, skin cosmetics, hair cosmetics, washes). Examples of such components include surfactants (such as emulsifiers), oily components, alcohols, solubilizers, thickeners, antioxidants, chelating agents, pH adjusters, Flavoring agents, coloring matter, UV absorbers, UV-scattering agents, vitamins, amino acids, and preservatives.

Other moisturizers may be incorporated as long as the effects of the present invention are not impaired. Any moisturizer may be used, and examples of usable moisturizers include polyhydric alcohols, such as glycerol, propylene glycol, 1,3-butylene glycol, sorbitol, polyglycerols, polyethylene glycol, and dipropylene glycol; NMF components such as amino acids, sodium lactate, and sodium pyrrolidone carboxylate; and water-soluble high polymers, such as hyaluronic acid, collagen, mucopolysaccharides, and chondroitin sulfate.

The proportion of the skin moisturizer of the invention in the external preparation for the skin is not particularly limited, as long as the obtained external preparation for the skin has moisturizing effects attributable to the skin moisturizer of the invention. In general, the proportion can be appropriately selected from the range of 0.1 to 100 wt.%, preferably 1 to 100 wt.%, and more preferably 2.5 to 100 wt.%, based on the dry weight of the plant of the genus Litsea.

The form of the external preparation for the skin is not particularly limited. Examples of such forms include lotions (liquids, dispersions), emulsions, creams, ointments, aerosols, foams, patches such as poultices and packs, external pharmaceuticals, skin cosmetics, scalp cosmetics, and hair cosmetics (such as hair growth stimulants, hair conditioners), and washes (such as body washes, hair washes, and facial washes).

### (III) Blood Circulation Promoter

The pulverized leaves or a lipid-soluble fraction of a leaf extract of Litsea polyantha has excellent blood circulation-promoting effects as shown in Experiment Example 2 below, and therefore can be used as an active ingredient of blood circulation promoters that have blood flow-promoting effects. More specifically, the present invention can provide a blood circulation promoter in an epidermally administrable form containing pulverized leaves or a lipid-soluble fraction of a leaf extract of Litsea polyantha as an active ingredient.

The proportion of pulverized leaves or a lipid-soluble fraction of a leaf extract of Litsea polyantha in the blood circulation promoter of the invention is not particularly limited, as long as the obtained blood circulation promoter has blood flow-promoting effects. The proportion can be appropriately selected from the range of 0.1 to 100 wt.%, preferably 1 to 100 wt.%, and more preferably 2.5 to 100 wt.%, based on the total weight.

The form of the blood circulation promoter is not particularly limited, as long as the promoter is epidermally administrable. Examples of such forms include liquids, emulsions, creams, ointments, gels, aerosols, and patches such as packs.

The blood circulation promoter of the invention can also be used as an active ingredient of external preparations for the skin that have blood flow-promoting effects. Such an external preparation for the skin contains the blood circulation promoter as an essential ingredient, and optionally contains other components usually contained in external preparations for the skin (such as drugs, quasi drugs, skin cosmetics, scalp cosmetics, and washes). Examples of such components include surfactants (such as emulsifiers), oily components, alcohols, solubilizers, thickeners, antioxidants, chelating agents, pH adjusters, Flavoring agents, coloring matters, UV absorbers, UV-scattering agents, vitamins, amino acids, and preservatives.

Other blood circulation promoters may be incorporated, as long as the effects of the invention are not impaired. Any blood circulation promoter may be used, and examples of promoters that can be incorporated include capsicum tincture, γ-orizanol, and the like.

The proportion of the blood circulation promoter of the invention in the external preparation for the skin is not particularly limited, as long as the obtained external preparation for the skin has blood circulation-promoting effects attributable to the blood circulation promoter of the invention. In general, the proportion can be appropriately selected from the range of 0.1 to 100 wt.%, preferably 1 to 100 wt.%, and more preferably 2.5 to 100 wt.%, based on the dry weight of the leaves of Litsea polyantha.

The form of the external preparation for the skin is not particularly limited. Examples of such forms include lotions (liquids, dispersions), emulsions, creams, ointments, aerosols, foams, patches such as poultices, and packs, external pharmaceuticals, skin cosmetics, scalp cosmetics, and hair cosmetics (such as hair growth stimulants and hair conditioners), and washes (such as body washes, hair washes and facial washes). The external skin preparation, which contains the blood flow promoter of the invention as an active ingredient, can promote blood flow in the face, head, limbs or the whole body to thereby prevent or ameliorate disorders (such as skin dullness, age spots, wrinkles, hair loss, and thinning hair due to reduced metabolism) and pathologic conditions (such as suffering from sensitivity to cold due to poor circulation, stiff shoulders associated with poor circulation, congestion, bedsores, Raynaud's disease, and economy-class syndrome), all caused by blood circulation disorders.

### EXAMPLES

The following Experimental Examples serve to illustrate the invention in more detail, without limiting the scope of the invention. In the Examples, all percentages are by weight unless otherwise specified.

### Reference Experiment Examples

### (I) Measurement of Electrical Activity in the Cutaneous Arterial Sympathetic Nerve (Efferent Branch)

### (1) Experimental Method

### (1-1) Preparation of Test Samples

Urea was dissolved in an equal amount of water, and the resulting solution was mixed with a base cream (ingredients: dipropylene glycol, cetanol, 1,3-butylene glycol, stearyl alcohol, glyceryl stearate, stearic acid, polyethylene glycol-75, steareth-20, ceteth-20, jojoba oil, hydrogenated polyisobutene, ethoxydiglycol behenate, phenoxyethanol, ethylparaben, and methylparaben) to prepare a cream containing 10 wt.% of urea (a urea-containing cream).

### (1-2) Experimental Subjects

Male Wister rats housed under a constant cycle of 12 hours of light (lights on from 7:00 to 19:00 at 80 lux) and 12 hours of darkness (19:00 to 7:00) for 1 week (body weight: 250 to 300 g) were used as experimental subjects (non-human subjects). Before the experiment, food ("MF" type; product of Oriental Yeast) and water were freely available to the rats.

To prevent the effect of smell stimulus on the sympathetic nerve, every day, starting from five days before the experiment, a 1% aqueous zinc sulfate solution was applied to the nasal mucosa of each experimental subject under pentobarbital anesthesia (30mg/kg) and allowed to stand for 10 minutes for anosmia treatment (Kolunie JM. Stern JM: Horm Behav 1995, 29:492-518).

After the rats were subjected to fasting for 6 hours, 2 g of the urea-containing cream or the base cream as a control was applied to the tail of each rat under urethane anesthesia (with an aqueous solution of urethane being intraperitoneally administered in an amount of 1 g/kg of body weight). The changes in nerve activity (electrical activity) of the efferent branches of the cutaneous arterial sympathetic nerves were measured for 60 minutes, immediately after application.

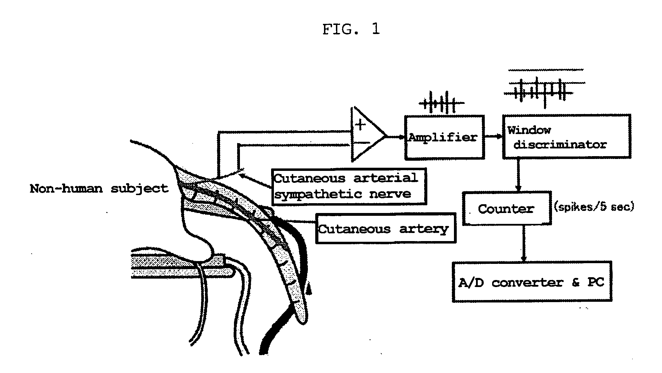

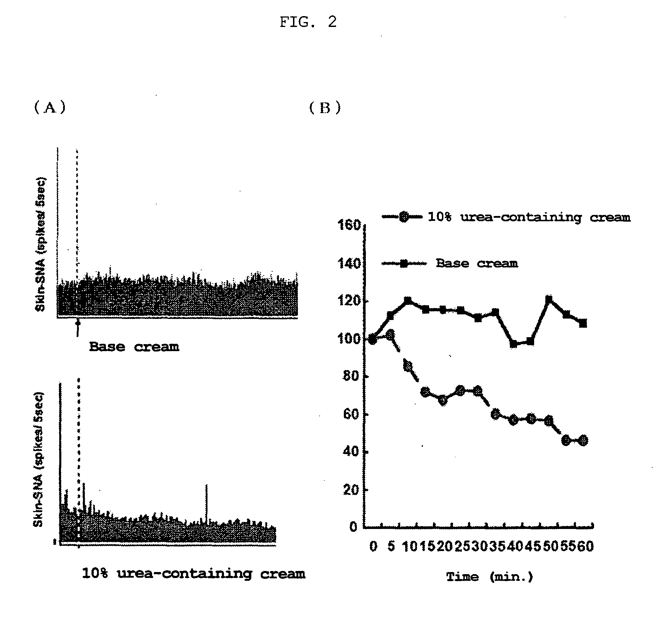

More specifically, the measurement of electrical activity in the cutaneous arterial sympathetic nerve (efferent branch) was performed by hooking up efferent branch of cutaneous arterial sympathetic nerve fibers of each rat with a pair of silver-wire electrodes under a stereoscopic microscope. To prevent drying, the electrodes were immersed in a mixture of liquid paraffin and a mixure of warm vaseline. The obtained electrical activity in the nerve was amplified with a differential amplifier and monitored using an oscilloscope. To detect nerve activity in the cutaneous arterial sympathetic nerve, noise signals were separated by a window discriminator, and converted to spike signals. The obtained spike signals were counted, based on the number of spikes per 5 seconds, using a rate meter, then subjected to A/D (analog/digital) conversion, and recorded on a personal computer (see FIG. 1). The electrical activity was recorded for 60 minutes (see FIG. 2B).

### (2) Experimental Results

FIG. 2 shows the results. FIG. 2 (A) shows the measurement data, and FIG. 2 (B) is a graph of the measurement data plotted with the data measured before application of the cream being defined as 100%. The upper row of FIG. 2 (A) shows the measurement data from the rat to which the base cream (control) was applied (shown as -■- in FIG. 2 (B)). The lower row of FIG. 2 (A) shows the measurement data from the rat to which the urea-containing cream was applied (shown as -●- in FIG. 2 (B)).

FIG. 2 (A) and (B) clearly show that when the base cream (control) was applied to the tail of the rat, activity (electrical activity) in the cutaneous arterial sympathetic nerve hardly changed compared to the activity measured before application of the base cream; in contrast, when the urea-containing cream was applied to the tail of the rat, activity in the cutaneous arterial sympathetic nerve was significantly reduced.

The above results show that application of a 10% urea-containing cream to the skin reduces activity in the sympathetic nerve that controls the cutaneous artery. In contrast, when the base cream was applied to the tail of rat (control rat), no substantial change was observed in the nerve activity (electrical activity) of the skin sympathetic nerve, compared to the activity before application of the cream, and such a reduction as seen in the test rat was not observed.

### (II) Measurement of Trans-Epidermal Water Loss

160 mg of the 10% urea-containing cream prepared in Reference Experimental Example (I) was applied to a skin area of 2 x 6 cm (12 cm²) on the left side of the back of male HWY (Hairless Wistar Yagi) rats weighing about 250 g, without using anesthesia, whereas 160 mg of a base cream as a control was applied to a skin area of 2 x 6 cm (12 cm²) on the right side of the back of the same. Eighteen hours and twenty-four hours after application, a portable evaporimeter ("VapoMeter", product of Delfin Technologies Ltd) was pressed against each skin area thus treated to measure the transepidermal water loss (TEWL).

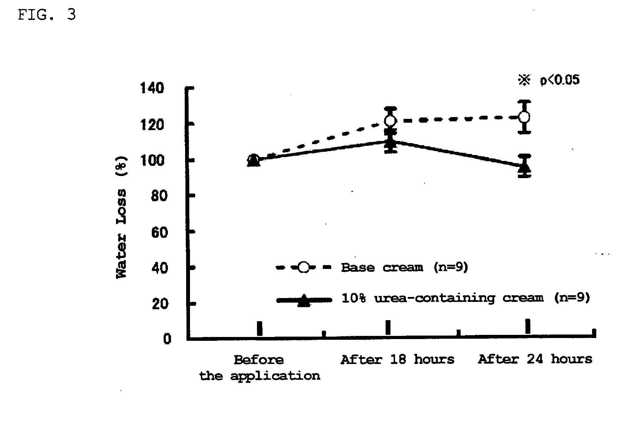

Figure 3 shows the results. The results are expressed as the relative transepidermal water loss (%) measured 18 hours and 24 hours after application, with the transepidermal water loss before application being defined as 100%.

### Consideration

The experimental results confirmed that the application of a cream containing 10% of urea known as a moisturizer (urea-containing cream) to the skin reduces the transepidermal water loss (Experiment (II)) and concurrently reduces activity (electrical activity) in the cutaneous arterial sympathetic nerve. The results show that there is a correlation between the moisturizing effect on the skin and activity (electrical activity) in the cutaneous arterial sympathetic nerve, so that by measuring the activity (electrical activity) in the cutaneous arterial sympathetic nerves and using the reduction of such activity as an index, the moisturizing effects of test substances on the skin can be evaluated.

### Experiment Example 1 Evaluation of the Moisturizing Effects of Extracts of a Plant of the Genus Litsea

The electrical activity in cutaneous arterial sympathetic nerves (efferent branches) and the transepidermal water loss were measured according to the methods described in (I) and (II) of the Reference Experimental Examples, using as test samples pulverized leaves and solvent extracts of the leaves of Litsea polyantha (hereinafter simply referred to as "Litsea"), that is a plant belonging to the genus Litsea of the family Lauraceae.

### (1) Preparation of Test Samples

Two grams each of a base cream (ingredients: dipropylene glycol, cetyl alcohol, 1,3-butylene glycol, stearyl alcohol, glyceryl stearate, stearic acid, polyethylene glycol-75, steareth-20, ceteth-20, Jojoba oil, hydrogenated polyisobutene, ethoxydiglycol behenate, phenoxyethanol, ethylparaben, and methylparaben) was mixed with each of the pulverized leaves of Litsea and the solvent extracts of the leaves of Litsea to prepare creams containing Litsea (Litsea-containing creams) as test samples.

A lipid-soluble fraction and a water-soluble fraction obtained in the methods described below were used as the solvent extracts.

### Preparation of Solvent Extracts

(a) Lipid-soluble fraction: 1 ml of water, 2.5 ml of methanol, and 1.25 ml of chloroform were added to 50 mg of Litsea leaves. After the mixture was allowed to stand at room temperature for 10 minutes, 1.25 ml of chloroform was added, and the mixture was fully stirred in a vortex. Further, 1.25 ml of water was added thereto (to achieve a final water:methanol:chloroform ratio of 0.9:1:1). After the mixture was centrifuged, the lower chloroform layer was collected and air dried on a Petri dish. The contents of the Petri dish were dissolved in 0.5 ml of chloroform and used as the lipid-soluble fraction.
(b) Water-soluble Fraction: The upper water layer (about 3 ml) obtained by centrifugation was evaporated to dryness using a centrifugal concentrator ("CC-101", product of Tomy Seiko Co., Ltd.). The resulting product was dissolved in 0.5 ml of water and used as the water-soluble fraction.

Each of the Lipid-soluble fraction and the water-soluble fraction was mixed with 2 g each of the base cream, and used as test samples (a lipid-soluble fraction sample and a water-soluble fraction sample).

### (2) Experimental Results

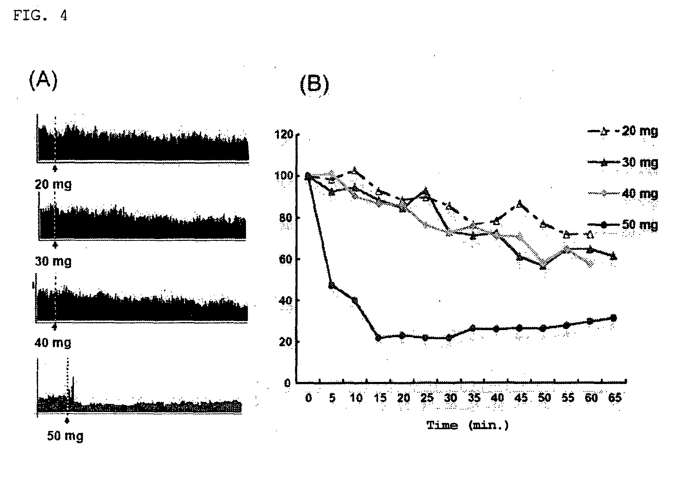

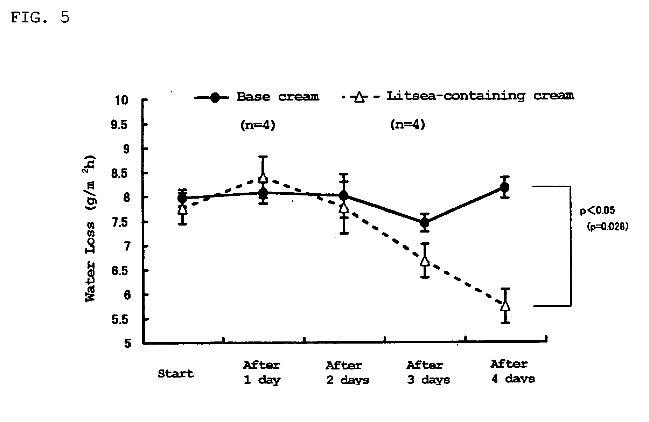

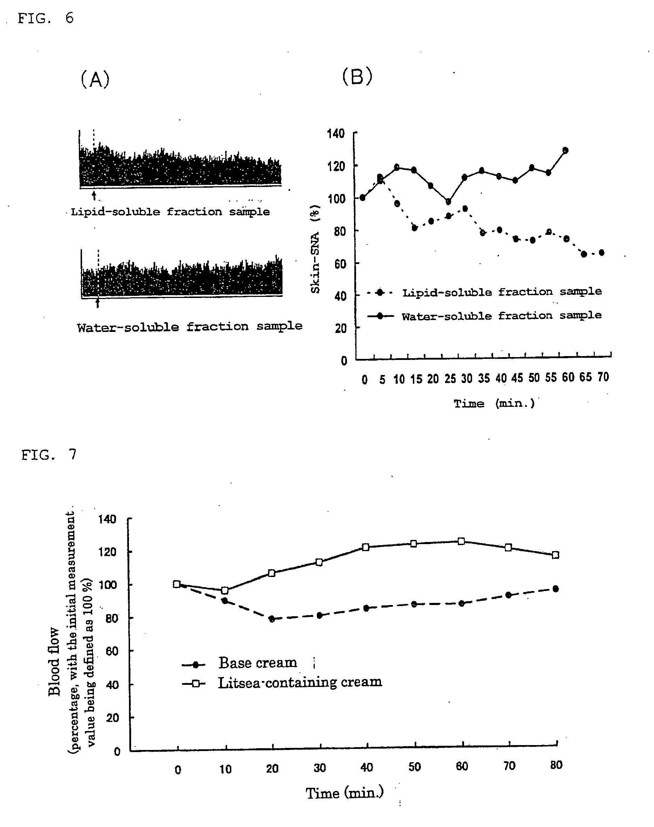

(2-1) FIG. 4 shows the results of measurement of the electrical activity in the cutaneous arterial sympathetic nerve obtained when Litsea-containing creams containing pulverized leaves of Litsea (20 to 50 mg/2 g) were applied to the skin. FIG. 5 shows the results of measurement transepidermal water loss obtained when a Litsea-containing cream (50 mg/2 g, a cream containing 2.5% pulverized leaves of Litsea) was applied to the skin. FIG. 4 (A) shows the measured data, and FIG. 4 (B) is a graph of the measured data with the value measured before application of the cream being defined as 100%. In FIG. 5, the solid line (-●-) shows the results in the rats to which the base cream (control) was applied, and the dashed line (--Δ--) shows the results in the rats to which the Litsea-containing cream was applied.
   These results clearly show that when the base cream (control) was applied to the tails of the rats, the activity (electrical activity) in the cutaneous arterial sympathetic nerve hardly changed, compared to that before application of the base cream; in contrast, when the Listea-containing cream was applied to the tails of the rats, the activity of the cutaneous arterial sympathetic nerve was significantly reduced. The application of the Litsea-containing cream to the skin reduced the activity of the sympathetic nerves that control the cutaneous arteries (FIG. 4) and concurrently reduced the transepidermal water loss (FIG. 5). In contrast, no inhibition of transepidermal water loss was observed in the rats (control group) to which the base cream was applied.
   The results show that Litsea, which is a plant belonging to the genus Litsea of the family Lauraceae, has an effect of inhibiting water loss from the skin and thereby enhancing the moisture retention of the skin (moisturizing effect).
(2-2) FIG. 6 shows the results of measurement of the electrical activity in the cutaneous arterial sympathetic nerves obtained by applying Litsea-containing creams (water-soluble fraction sample and Lipid-soluble fraction sample) containing the extracts (water-soluble fraction and lipid-soluble fraction) of the leaves of Litsea prepared according to the method described in the section of Preparation of Test Samples. In FIG. 6, the solid line (-●-) shows the results for the water-soluble fraction of the leaves of Litsea, whereas the dashed line (--●--) shows the results for the lipid-soluble fraction of the leaves of Litsea. FIG. 6 (A) shows the measured data, and FIG. 6 (B) is a graph of the measured data with the value measured before application of the cream being defined as 100%.

These results clearly show that when the cream containing the lipid-soluble fraction of Litsea was applied to the tails of the rats, the activity in the cutaneous arterial sympathetic nerve was reduced; in contrast, the application of the water-soluble fraction of Litsea did not reduce the activity (electrical activity) in the cutaneous arterial sympathetic nerve. These results suggest that the lipid-soluble fraction of Litsea is a fraction with skin-water retention activity, and the fraction contains an active ingredient for producing moisturizing effects.

### Experiment Example 2 Evaluation of the Blood Flow-Promoting Effects of Extracts of a Plant of the Genus Litsea

Two grams of the Litsea-containing cream containing pulverized leaves of Litsea (50 mg/2 g)(see Experiment Example 1) or the base cream (control) was applied to the tails and hairless portions of male Wistar rats (body weight: about 350 g). A tip of a laser blood flowmeter ("ADVANCE LASER FLOWMETER ALF21", product of Advance) was attached to a fixed point of the stem of the tail using surgical tape, and the blood flow was measured. The values measured in terms of ml/min/100 g of the tissue were expressed as percentages, with the measured value before the cream application being defined as 100%. The absolute values, that are the 0 min values, were in the range of 3 to 5 ml/min/100 g of the tissue.

FIG. 7 shows the results. In FIG. 7, the solid line (-□-) shows the results obtained when the Litsea-containing cream was applied, and the dashed line (--●--) shows the results obtained when the base cream was applied. These results show that the epidermal administration of the Litsea-containing cream promotes blood flow in the skin.

### Consideration

Cutaneous arteries are controlled by the sympathetic nerve, and noradrenaline emitted from sympathetic nerve endings constricts cutaneous blood vessels via α1 and α2 adrenergic receptors (W. F. Ganon, Review of Medical Physiology, Lange Medical Books, pp. 227, 2005). In contrast, there is no report disclosing that the cutaneous arteries are controlled by parasympathetic nerves. Thus, if the sympathetic nerve activity that controls a cutaneous artery is reduced, the cutaneous blood vessel is expanded so that blood flow increases. The results obtained in Experimental Examples 1 and 2 exactly reflect this biological reaction. More specifically, the results show that Litsea is capable of reducing cutaneous sympathetic nerve activity and, thus, has blood flow-increasing (blood circulation-promoting) effects. It is considered that the moisturizing effects of Litsea demonstrated in Experiment Example 1 were achieved because increased blood flow increased the supply of oxygen and nutrients to skin cells.

### Formulation Examples

Embodiments of external preparations for skin of the present invention are described below. The lipid-soluble fraction of Litsea leaves obtained in Experimental Example 2 was used as the "processed product of a plant of the genus Litsea".

### Formulation Example 1 Emulsion

| | |
|---|---|
| 1. Squalane | 10.0 (wt.%) |
| 2. Methylphenylpolysiloxane | 4.0 |
| 3. Hydrogenated Palm Kernel Oil | 0.5 |
| 4. Hydrogenated Soybean Phospholipid | 0.1 |
| 5. Polyoxyethylene Sorbitan Monostearate (20E.O.) | 1.3 |
| 6. Sorbitan Monostearate | 1.0 |
| 7. Glycerol | 4.0 |
| 8. Methyl para-Hydroxybenzoate | 0.1 |
| 9. Carboxyvinyl polymer | 0.15 |
| 10. Purified Water | 56.35 |
| 11. Arginine (1 wt.% aqueous solution) | 20.0 |
| 12. Processed Product of a Plant of the Genus Litsea | 2.5 |

Production Process: Heat and dissolve oil-phase Components 1 to 6 at 80°C. Heat and dissolve water-phase Components 7 to 10 at 80°C. Add the oil-phase Components to the water-phase Components, while stirring. Uniformly emulsify the mixture using a homogenizer. Start cooling after the emulsification. Add Components 11 and 12 sequentially and mix uniformly.

### Formulation Example 2 Lotion

| | |
|---|---|
| 1. Ethanol | 15.00 (wt.%) |
| 2. Polyoxyethylene (40E.O.) Hydrogenated Castor Oil | 0.30 |
| 3. Flavoring agent | 0.10 |
| 4. Purified Water | 80.88 |
| 5. Citric Acid | 0.02 |
| 6. Sodium Citrate | 0.10 |
| 7. Glycerol | 1.00 |
| 8. Hydroxyethylcellulose | 0.10 |
| 9. Processed Product of a Plant of the Genus Litsea | 2.50 |

Production Process: Dissolve Components 2 and 3 in Component 1. After the dissolution, add Components 4 to 8 sequentially. After the mixture is fully stirred, add Component 9 and uniformly mix.

### Formulation Example 3 Cream

| | |
|---|---|
| 1. Squalane | 10.0 (wt.%) |
| 2. Stearic Acid | 2.0 |
| 3. Hydrogenated Palm Kernel Oil | 0.5 |
| 4. Hydrogenated Soybean Phospholipid | 0.1 |
| 5. Cetyl Alcohol | 3.6 |
| 6. Lipid-soluble Glyceryl Monostearate | 2.0 |
| 7. Glycerol | 10.0 |
| 8. Methyl para-Hydroxybenzoate | 0.1 |
| 9. Arginine (aqueous 20 wt.% solution) | 15.0 |
| 10. Purified Water | 39.2 |
| 11. Carboxyvinyl Polymer (aqueous 1 wt.% solution) | 15.0 |
| 12. Processed Product of a Plant of the Genus Litsea | 2.5 |

Process: Heat and dissolve oil-phase Components at 80°C. Heat and add water-phase Components 7 to 10 at 80°C. Add the oil-phase Components to the water-phase Components, while stirring. Uniformly emulsify the mixture using a homogenizer. After the emulsification, add Component 11 and start cooling. Add Component 12 at 40°C and uniformly mix.

### Formulation Example 4 Essence

| | |
|---|---|
| 1. Purified Water | 25.95 (wt.%) |
| 2. Glycerol | 10.0 |
| 3. Sucrose Fatty Acid Ester | 1.3 |
| 4. Carboxyvinyl Polymer (aqueous 1 wt.% solution) | 17.5 |
| 5. Sodium Alginate (aqueous 1 wt.% solution) | 15.0 |
| 6. Polyglyceryl Monolaurate | 1.0 |
| 7. Macadamia Nut Oil Fatty Acid Phytosteryl | 3.0 |
| 8. Di(phytosteryl-2-octyldodecyl) N-lauroyl-L-glutamate | 2.0 |
| 9. Hardened Palm Oil | 2.0 |
| 10. Squalane (derived from Olive) | 1.0 |
| 11. Behenyl Alcohol | 0.75 |
| 12. Beeswax | 1.0 |
| 13. Jojoba Oil | 1.0 |
| 14. 1,3-butylene Glycol | 14.0 |
| 15. L-Arginine (aqueous 10 wt.% solution) | 2.0 |
| 16. Processed Product of a Plant of the Genus Litsea | 2.5 |

Production Process: Mix water-phase Components 1 to 6 and heat and dissolve the mixture at 75°C. Mix oil-phase Components 7 to 14 and heat and dissolve the mixture at 75°C. Add the oil-phase Components to the water-phase Components. After the mixture is preliminarily emulsified, uniformly emulsify the mixture using a homomixer. After the emulsification, start cooling and add Component 15 at 50°C. After the mixture is cooled to 40°C, add Component 16 and uniformly mix.

### Formulation Example 5 Aqueous Gel

| | |
|---|---|
| 1. Carboxyvinyl Polymer | 0.5 (wt.%) |
| 2. Purified Water | 86.7 |
| 3. Sodium Hydroxide (aqueous 10 wt.% solution) | 0.5 |
| 4. Ethanol | 10.0 |
| 5. Methyl para-Hydroxybenzoate | 0.1 |
| 6. Flavoring agent | 0.1 |
| 7. Processed Product of a Plant of the Genus Litsea | 2.0 |
| 8. Polyoxyethylene (60E.O.) Hydrogenated Castor Oil | 0.1 |

Production Process: Add Component 1 to Component 2 and uniformly stir the mixture. Add Component 3 and uniformly stir the mixture. Add Component 5, which has been dissolved in Component 4 beforehand. After the mixture is uniformly stirred, add Components 6 to 8, which have been mixed beforehand. Uniformly stir and mix the mixture.

### Formulation Example 6 Cleansing Wash

| | |
|---|---|
| 1. Squalane | 81.0 (wt.%) |
| 2. Polyoxyethylene Glyceryl Isostearate | 15.0 |
| 3. Purified Water | 3.0 |
| 4. Processed Product of a Plant of the Genus Litsea | 1.0 |

Production Process: Uniformly dissolve Component 1 in Component 2. Add Components 3 and 4 sequentially and uniformly mix.

### Formulation Example 7 Cleansing Foam

| | |
|---|---|
| 1. Stearic Acid | 16.0 (wit.%) |
| 2. Myristic Acid | 16.0 |
| 3. Lipid-soluble Glyceryl Monostearate | 2.0 |
| 4. Glycerol | 20.0 |
| 5. Sodium Hydroxide | 7.5 |
| 6. Palm Oil Fatty Acid Amide Propylbetaine | 1.0 |
| 7. Purified Water | 36.5 |
| 8. Processed Product of a Plant of the Genus Litsea | 1.0 |

Production Process: Heat and dissolve oil-phase Components 1 to 4 at 80°C. Heat and dissolve water-phase Components 5 to 7 at 80°C. Uniformly mix and stir the oil-phase components and the water-phase components. Start cooling. Add Component 8 at 40°C and uniformly mix.

### Formulation Example 8 Water-in-Oil Emollient Cream

| | |
|---|---|
| 1. Liquid Paraffin | 30.0 (wt.%) |
| 2. Microcrystalline Wax | 2.0 |
| 3. Vaseline | 5.0 |
| 4. Diglycerol Oleate | 5.0 |
| 5. Sodium Chloride | 1.3 |
| 6. Potassium Chloride | 0.1 |
| 7. Propylene Glycol | 3.0 |
| 8. 1,3-Butylene Glycol | 5.0 |
| 9. Methyl para-Hydroxybenzoate | 0.1 |
| 10. Processed Product of a Plant of the Genus Litsea | 2.5 |
| 11. Purified Water | 45.9 |
| 12. Flavoring agent | 0.1 |

Production Process: Dissolve Components 5 and 6 in a portion of Component 11 and adjust the solution temperature to 50°C. While stirring, gradually add this solution to Component 4, which has been heated to 50°C. After the mixture is fully mixed, uniformly disperse the mixture in Components 1 to 3, which have been heated and dissolved at 70°C. While stirring, add to this dispersion Components 7 to 10, which have been heated and dissolved in the rest of Component 11 at 70°C. Emulsify the mixture using a homomixer. Start cooling after the emulsification. Add Component 12 at 40°C and uniformly mix.

### Formulation Example 9 Pack

| | |
|---|---|
| 1. Purified Water | 63.9 (wt.%) |
| 2. Polyvinyl Alcohol | 12.0 |
| 3. Ethanol | 17.0 |
| 4. Glycerol | 5.0 |
| 5. Polyethylene Glycol (Average Molecular Weight: 1000) | 2.0 |
| 6. Processed Product of Litsea Genus Plant | 0.1 |

Production Process: Mix Components 2 and 3 and heat the mixture to 80°C. Dissolve the mixture in Component 1, which has been heated to 80°C. After the mixture is uniformly dissolved, add Components 4 and 5 and start cooling, while stirring. When the mixture has been cooled to 40°C, add Components 6 and 7 and uniformly mix.

### Formulation Example 10 Bath Essence

| | |
|---|---|
| 1. Flavoring agent | 0.3 (wt.%) |
| 2. Processed Product of a Plant of the Genus Litsea | 1.0 |
| 3. Sodium Bicarbonate | 50.0 |
| 4. Sodium Sulfate | 48.7 |

Production Process: Uniformly mix Components 1 to 4.

### Formulation Example 11 Hair Wax

| | |
|---|---|
| 1. Stearic Acid | 3.0 (wt.%) |
| 2. Microcrystalline Wax | 2.0 |
| 3. Cetyl Alcohol | 3.0 |
| 4. Highly Polymerized Methyl Polysiloxane | 2.0 |
| 5. Methyl Polysiloxane | 5.0 |
| 6. Poly(oxyethylene-oxypropylene)methylpolysiloxane Copolymer | 1.0 |
| 7. Methyl para-Hydroxybenzoate ' | 0.1 |
| 8. 1,3-Butylene Glycol | 7.5 |
| 9. Arginine | 0.7 |
| 10. Purified Water | 74.6 |
| 11. Processed Product of a Plant of the Genus Litsea | 1.0 |
| 12. Flavoring agent | 0.1 |

Production Process: Mix oil-phase Components 1 to 6 and heat and dissolve the mixture at 75°C. Mix water-phase Components 7 to 10 and heat and dissolve the mixture at 75°C. Add the oil-phase components to the water-phase components. Emulsify the mixture using a homomixer. Start cooling after the emulsification. Add Components 11 and 12 at 40°C and uniformly mix.

### Formulation Example 12 Hair Tonic

| | |
|---|---|
| 1. Ethanol | 50.0 (wt.%) |
| 2. Purified Water | 48.9 |
| 3. Processed Product of a Plant of the Genus Litsea | 1.0 |
| 4. Flavoring agent | 0.1 |

Production Process: Uniformly mix Components 1 to 4.

### BRIEF DESCRIPTION OF THE DRAWINGS

FIG. 1 is a schematic diagram of the process of measuring electrical activity in a cutaneous arterial sympathetic nerve used in the present invention. The diagram shows an example in which epidermal application to the tail is used as the administration method and an efferent branch of the cutaneous arterial sympathetic nerve of the tail is used as the site of the measurement of electrical activity.
FIG. 2 (A) shows changes over time (the measured data) of the activity (electrical activity) in the efferent branch of the cutaneous arterial sympathetic nerve, when the base cream (upper column) or the urea-containing cream (lower column) was applied to the skin. FIG. 2 (B) is a graph of the measured data (Experimental Example 1), with the electrical activity in the cutaneous arterial sympathetic nerve being plotted on the ordinate, and the electrical activity before application of each cream being defined as 100%.
FIG. 3 shows the water loss (%), when the base cream (-○-) or the urea-containing cream (-▲-) was applied to the back of hairless rats. The water loss (%) was expressed in terms of percentage, with the water loss before application of each cream being defined as 100% (the same applied hereinafter).
FIG. 4 shows the results of the measurement of electrical activity in the cutaneous arterial sympathetic nerve, when a cream containing pulverized leaves of Litsea (20 to 50 mg/2 g) was applied. FIG. 4 (A) shows the measurement data, and FIG. 4 (B) is a graph of the measurement data, with the electrical activity of the cutaneous arterial sympathetic nerve being plotted on the ordinate, and the electrical activity before application of each cream being defined as 100% (Experimental Example 1).
FIG. 5 shows the water loss (%) when the base cream (-●-) or the cream containing pulverized leaves of Litsea (-△-) (50 mg/2 g) was applied to the back of hairless rats (Experimental Example 1).
FIG. 6 shows the results of the measurement of electrical activity in the cutaneous arterial sympathetic nerves, when the cream containing a water-soluble fraction of Litsea leaves or the cream containing a lipid-soluble fraction of Litsea leaves was applied. FIG. 6 (A) shows the measurement data, and FIG. 6 (B) shows a graph of the measurement data with the electrical activity of the cutaneous arterial sympathetic nerve being plotted on the ordinate, and the electrical activity before application of each cream being defined as 100% (Experimental Example 1).
FIG. 7 shows changes in the skin blood flow, when 2 g each of the cream containing pulverized leaves of Litsea (50 mg/2 g) and the base cream (control) were applied to the skin. The blood flow (ml/min /100 g of the tissue) was plotted on the ordinate, and expressed in terms of percentage, with the initial measurement value being defined as 100%.

## Claims

1. A skin moisturizer comprising pulverized leaves or a lipid-soluble fraction of a leaf extract of Litsea polyantha in an amount effective to reduce the sympathetic nerve activity of a cutaneous artery.

2. A blood circulation promoter comprising pulverized leaves or a lipid-soluble fraction of a leaf extract of Litsea polyantha in an amount effective to reduce the sympathetic nerve activity of a cutaneous artery.

3. An external preparation for the skin comprising pulverized leaves or a lipid-soluble fraction of a leaf extract of Litsea polyantha in an amount effective to reduce the sympathetic nerve activity of a cutaneous artery.

4. An external preparation for the skin containing a skin moisturizer described in claim 1 or a blood circulation promoter described in claim 2 in an amount effective to reduce the sympathetic nerve activity of a cutaneous artery.

5. Use of pulverized leaves or a lipid-soluble fraction of a leaf extract of Litsea polyantha in production of a skin moisturizer.

6. Use of pulverized leaves or a lipid-soluble fraction of a leaf extract of Litsea polyantha in production of an external skin preparation for promoting blood flow in the face, head, limbs or the whole body.

## Patentansprüche

1. Hautfeuchthaltemittel, welches pulverisierte Blätter oder eine fettlösliche Fraktion eines Blattextrakts von *Litsea polyantha* in einer zur Reduzierung der sympathischen Nervenaktivität einer kutanen Arterie wirksamen Menge umfasst.

2. Mittel zur Anregung des Blutkreislaufs, welches pulverisierte Blätter oder eine fettlösliche Fraktion eines Blattextrakts von *Litsea polyantha* in einer zur Reduzierung der sympathischen Nervenaktivität einer kutanen Arterie wirksamen Menge umfasst.

3. Zubereitung zur äußeren Anwendung für die Haut, welche pulverisierte Blätter oder eine fettlösliche Fraktion eines Blattextrakts von *Litsea polyantha* in einer zur Reduzierung der sympathischen Nervenaktivität einer kutanen Arterie wirksamen Menge umfasst.

4. Zubereitung zur äußeren Anwendung für die Haut, welche ein Hautfeuchthaltemittel nach Anspruch 1 oder ein Mittel zur Anregung des Blutkreislaufs nach Anspruch 2 in einer zur Reduzierung der sympathischen Nervenaktivität einer kutanen Arterie wirksamen Menge enthält.

5. Verwendung von pulverisierten Blättern oder einer fettlöslichen Fraktion eines Blattextrakts von *Litsea polyantha* zur Herstellung eines Hautfeuchthaltemittels.

6. Verwendung von pulverisierten Blättern oder einer fettlöslichen Fraktion eines Blattextrakts von *Litsea polyantha* zur Herstellung einer Hautzubereitung zur äußeren Anwendung, um den Blutfluss im Gesicht, Kopf, Gliedmaßen oder dem ganzen Körper anzuregen.

## Revendications

1. Hydratant pour la peau comprenant des feuilles réduites en poudre ou une fraction soluble dans les lipides d'un extrait de feuille de Litsea polyantha en une quantité efficace pour réduire l'activité du nerf sympathique d'une artère cutanée.

2. Promoteur de la circulation sanguine comprenant des feuilles réduites en poudre ou une fraction soluble dans les lipides d'un extrait de feuille de Litsea polyantha en une quantité efficace pour réduire l'activité du nerf sympathique d'une artère cutanée.

3. Préparation externe pour la peau comprenant des feuilles réduites en poudre ou une fraction soluble dans les lipides d'un extrait de feuille de Litsea polyantha en une quantité efficace pour réduire l'activité du nerf sympathique d'une artère cutanée.

4. Préparation externe pour la peau comprenant un hydratant pour la peau décrit à la revendication 1 ou un promoteur de la circulation sanguine décrit à la revendication 2 en une quantité efficace pour réduire l'activité du nerf sympathique d'une artère cutanée.

5. Utilisation de feuilles réduites en poudre ou d'une fraction soluble dans les lipides d'un extrait de feuille de Litsea polyantha dans la production d'un hydratant pour la peau.

6. Utilisation de feuilles réduites en poudre ou d'une fraction soluble dans les lipides d'un extrait de feuille de Litsea polyantha dans la production d'une préparation externe pour la peau destinée à favoriser le flux sanguin dans le visage, dans la tête, dans les membres ou dans tout le corps.
